(19) Europäisches Patentamt
European Patent Office
Office européen des brevets

(11) EP 4 273 209 A1

(12) EUROPEAN PATENT APPLICATION

(43) Date of publication:
**08.11.2023 Bulletin 2023/45**

(21) Application number: **22171692.1**

(22) Date of filing: **04.05.2022**

(51) International Patent Classification (IPC):
***C11D 3/386*** *(2006.01)*

(52) Cooperative Patent Classification (CPC):
**C12N 9/88; C11D 3/38636; C11D 11/0041; C12Y 402/02; C12Y 402/02003; C12Y 402/02011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company**
**Cincinnati, OH 45202 (US)**

(72) Inventors:
- **JONES, Catherine**
  **Newcastle upon Tyne, NE1 7RU (GB)**
- **LANT, Neil Joseph**
  **Newcastle upon Tyne, NE12 9BZ (GB)**
- **MOMIN, Nazarmohammad Gulamhussain**
  **Newcastle upon Tyne, NE12 9BZ (GB)**
- **MORALES GARCIA, Ana L.**
  **Newcastle upon Tyne, NE12 9BZ (GB)**
- **VALENTINI, Alessandra**
  **Newcastle upon Tyne, NE12 9BZ (GB)**
- **WILLATS, William G. T.**
  **Newcastle upon Tyne, NE15 0NS (GB)**
- **YAU, Hamish Chun Lam**
  **Newcastle upon Tyne, NE12 9BZ (GB)**

(74) Representative: **P&G Patent Belgium UK**
**N.V. Procter & Gamble Services Company S.A.**
**Temselaan 100**
**1853 Strombeek-Bever (BE)**

Remarks:
The complete document including Reference Table(s) and the Sequence Listing(s) can be downloaded from the EPO website

(54) **MACHINE-CLEANING COMPOSITIONS CONTAINING ENZYMES**

(57) A washing machine-cleaning composition comprising (a) at least two enzymes selected from (i) alginate lyase enzyme; (ii) Pel-ase enzyme; (iii) Psl-ase enzyme; (iv) β-1,3- glucanase enzyme; and (v) β-1,3(4)- glucanase enzyme; and (b) a cleaning adjunct and a method of treating the interior of a washing machine and a method of degrading soils on an interior surface of an washing machine using such a composition.

EP 4 273 209 A1

## Description

REFERENCE TO A SEQUENCE LISTING

This application contains a Sequence Listing in computer readable form. The computer readable form is incorporated herein by reference.

FIELD OF THE INVENTION

The present invention relates to a machine-cleaning composition, which is particularly suitable for use in a dishwashing or laundry washing machine, particularly a laundry washing machine. The invention also relates to use of the machine cleaner for cleaning dishwashing or laundry washing machines, particularly laundry washing machines.

BACKGROUND OF THE INVENTION

Over time soils build up in washing machines, particularly in areas where soils can become entrapped, for example internal surfaces of washing machines, filters, seals, drainage areas and/or on the laundry washing machine drum, particularly the outside surface. These soils can reduce efficacy of cleaning and create malodour. This problem is exacerbated by today's trend to colder, quicker washes to address environmental concerns, and also a reduction in cleaning chemistry such as bleaches. In these conditions, soils may deposit and build-up and this can even lead to proliferation of micro-organisms. The resulting deposited soils and especially soils produced by micro-organisms may be sticky, typically comprising for example, extracellular polymeric substance (EPS) which is a polymeric conglomeration generally composed of extracellular DNA, proteins, and polysaccharides. These soils can be particularly challenging to remove and may cause problems with the circulation of the wash and rinse waters leading to poor cleaning results including increased re-deposition of soils on the articles being washed and on the interior surfaces of the machine, and increased malodour.

It is well known in the art to use enzymes in compositions for cleaning dishwashing machines, for example as disclosed in WO 98/39402. However, there is still a need for improved means to ameliorate soil build-up inside washing machines such as dishwashing and laundry washing machines, particularly laundry washing machines, and to provide cleaning for such machines.

SUMMARY OF THE INVENTION

The present invention provides a washing machine-cleaning composition comprising (a) at least two enzymes selected from (i) alginate lyase enzyme; (ii) Pel-ase enzyme; (iii) Psl-ase enzyme; (iv) endo-β-1,3-glucanase enzyme; and (v) endo-β-1,3(4)-glucanase enzyme; and (b) a cleaning adjunct. Preferably the composition comprises from 0.00005 to 5 wt% alginate lyase enzyme (active enzyme protein), and/or from 0.00005 wt% to 5 wt% Pel-ase (active enzyme protein), and/or from 0.00005 to 5 wt% Psl-ase (active enzyme protein), and/or from 0.00005 wt% to 5 wt% enzyme (active enzyme protein) endo-β-1,3-glucanase enzyme and/or from 0.00005 wt% to 5 wt% enzyme (active enzyme protein) endo-β-1,3(4)-glucanase enzyme. Preferably the composition also comprises surfactant, preferably in an amount from 1 to 60 wt%.

The invention also provides a method of treating the interior of an automatic dishwashing machine and/or laundry washing machine to prevent or remove the build-up of soils, the method comprising the step of supplying the machine-cleaning composition to the interior of the machine during at least one wash cycle or at least one rinse cycle of the dishwashing machine or laundry washing machine. In particular, the method is for treating the interior of a laundry washing machine, where the soil build-up and the nature of the soil may be particularly problematic due to the combination of cooler washing temperatures and less harsh detergent compositions including more neutral pH conditions to minimize fabric damage from the laundering process.

The invention also provides a method of degrading soils on an interior surface of an automatic dishwashing machine or laundry washing machine, which method comprises the steps of providing the machine-cleaning composition during at least one or more wash cycles or one or more rinse cycles of the automatic dishwashing or laundry washing machine, the machine-cleaning composition being present in the wash or rinse water (aqueous wash liquor).

The invention also provides use of the machine-cleaning composition for cleaning washing machines such as laundry washing machines and/or dishwashing machines, either domestic or commercial/institutional, particularly for cleaning laundry washing machines.

The washing machine may be at least partially loaded with articles during the at least one wash cycle or at least one rinse cycle. The machine-cleaning composition may be used over two or more sequential wash cycles and/or rinse cycles of the washing machine.

The composition, method and use of the invention may also give rise to biofilm-disrupting effects and/or soil anti-redeposition effects.

DETAILED DESCRIPTION OF THE INVENTION

Definitions

Parent or Parent enzyme: The term "parent" or "parent enzyme" means an enzyme to which an alteration is made to produce the enzyme variants. The parent may be a naturally occurring (wild-type) polypeptide or a variant thereof.

Sequence Identity: The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

Alternatively, the parameters used may be gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100)/(\text{Length of Alignment} - \text{Total Number of Gaps in Alignment})$$

Variant: The term "variant" means a polypeptide having enzyme activity comprising an alteration/mutation, i.e., a substitution, insertion, and/or deletion, at one or more (e.g. several) positions relative to the parent. A substitution means a replacement of an amino acid occupying a position with a different amino acid; a deletion means removal of an amino acid occupying a position; and an insertion means adding 1-3 amino acids adjacent to and immediately following an amino acid occupying a position.

Wild-Type Enzyme: The term "wild-type" enzyme means an enzyme expressed by a naturally occurring micro-organism, such as a bacterium, algae, yeast, or filamentous fungus found in nature.

Washing Machine-Cleaning Composition

The washing machine-cleaning composition may be in liquid or solid form. It may be a liquid or gel, or powder or tablet or in a unit dose form, for example in the form of a liquid and/or solid, such as a powder, enclosed in a water-soluble film or water soluble sheet (such as a woven or non-woven fibrous sheet (unit dose pouch). Where the machine-cleaning composition is in the form of a unit dose pouch, the pouch may comprise a single or multiple compartments, or the composition may be divided between pouches so that the components of the composition are added by the addition of more than one pouch, the sum of the pouches to be used together in the washing machine adding all of the components of the machine-cleaning composition into the washing machine.

The washing machine-cleaning composition comprises (a) at least two enzymes selected from (i) alginate lyase enzyme; (ii) Pel-ase enzyme; (iii) Psl-ase enzyme; (iv) endo-β-1,3-glucanase enzyme; and (v) endo-β-1,3(4)-glucanase enzyme; and (b) a cleaning adjunct.

Alginate Lyase Enzyme

The alginate lyase is preferably microbial in origin, preferably bacterial or algal (for example from brown seaweed *(Phaeophyceae),* such as *Ascophyllum, Laminara, Macrocystis*), most preferably bacterial. The alginate lyase enzyme may be obtained from *Aeromonas sp., Azotobacter sp., Bacillus sp., Flavobacterium sp., Klebsiella sp., Pseudomonas sp., Sphingomonas sp., Vibrio sp., Zobellia galactanivorans*, most preferably *Flavobacterium sp.*

Preferably the alginate lyase enzyme comprises alginate lyase selected from: alginate lyase having at least

60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 1; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 3; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 7; or mixtures thereof. Preferred alginate lyase enzyme comprises alginate lyase enzyme corresponding to the wild-type or preferably a variant of the wild-type of any one of SEQ ID NOs: 1, 2, 3, 4, 5, 6 or 7 listed herein, or mixtures thereof. SEQ ID NOs 6 and 7 and variants thereof, and mixtures thereof are particularly preferred.

When the alginate lyase enzyme is a variant of a parent amino acid sequence, the parent alginate lyase enzyme preferably has a sequence identity to the polypeptide of one or more of SEQ ID NOs: 1, 2, 3, 4, 5, 6 or 7 of at least 50 % or at least 60%, or at least 70% or at least 80%, such as at least 85%, at least 90%, e.g. at least 92%, at least 93%, at least 94%, at least 95%, at least 96%, at least 97%, at least 98%, at least 99, or 100%, which has alginate lyase enzyme activity. It may be preferred for the variant amino acid sequence to differ from the parent alginate lyase by no more than fifteen, or no more than ten amino acids, or no more than five, or four or three or two or one amino acid(s) from the polypeptide of one or more of SEQ ID NOs: 1, 2, 3, 4, 5, 6 or 7.

When the alginate lyase enzyme is a variant of a parent amino acid sequence, the parent may be obtained from a microorganism of any genus. For purposes of the present invention, the term "obtained from" as used herein in connection with a given source shall mean that the parent encoded by a polynucleotide is produced by the source or by a cell in which the polynucleotide from the source has been inserted. In one aspect, the parent is secreted extracellularly. Variants may be prepared using any mutagenesis procedure known in the art, such as site-directed mutagenesis, synthetic gene construction, semi-synthetic gene construction, random mutagenesis, shuffling, etc.

The alginate lyase may be from any polysaccharide lyase (PL) family including PL5, PL6, PL7, PL14, PL15, PL17, PL18, PL32, PL34, and PL36. Preferably the alginate lyase is from PL family 7.

Preferably the alginate lyase enzyme has activity towards poly(beta-D-mannuronate) (polyM activity) and activity towards poly(alpha-L-guluronate) (polyG activity). The alginate lyase enzyme may comprise a single alginate lyase enzyme to provide the polyM activity and polyG activity or may comprise two or more alginate lyase enzymes which in combination provide the polyM and polyG activity. Preferably, the alginate lyase comprises an enzyme having both polyM activity and polyG activity. Preferably the polyM activity as defined by Test Method 1 in the test section herein, is at least 0.1 absorbance units, preferably at least 0.15 absorbance units and most preferably at least 2 absorbance units. Preferably the polyG activity as defined by Test Method 1 in the test section herein, is at least 0.3 absorbance units, preferably at least 0.4 absorbance units, or at least 0.5 or even at least 0.6 absorbance units. The alginate lyase enzyme may be incorporated into the cleaning compositions and methods of the invention in the form of a substantially pure enzyme. Alternatively, in particular where the enzyme is a variant of a wild-type enzyme, the variant is not recovered, but rather a host cell expressing the enzyme is used as the source of the alginate lyase enzyme.

The alginate lyase enzyme may be in the form of a liquid or a dry composition. For instance, the composition may be in the form of a granulate or a microgranulate. The alginate lyase enzyme may be stabilized including by encapsulation, in accordance with methods known in the art.

The alginate lyase enzyme is present in the composition in an amount from 0.00005 to 5 wt% active enzyme protein, preferably from 0.0001 to 2 wt% active protein or from 0.0005 or from 0.001 to 1 wt% active protein, or to 0.5 wt% or to 0.1 wt% or to 0.05 wt% active enzyme protein.

Preferably the alginate lyase enzyme is present in the aqueous wash liquor in an amount of from O.Olppm to 1000 ppm of the enzyme, or from 0.05 or from 0.1ppm to 750 or 500ppm.

Pel-ase Enzyme

The Pel-ase enzyme exhibits glycoside hydrolase activity towards Pel polysaccharide and preferably belongs to the endo-alpha-1,4-polygalactosminidase class (EC 3.2.1.109) of enzymes, preferably having at least 60% or 65% or more preferably at least 70% or at least 75% or at least 80% or at least 85% or at least 90% or at least 95% up to 100% identity to one or more of SEQ ID NOs: 8, 9, 10, 11, 12, 13, 14, 15, 16, 17, 18, 19, 20, 21, 22, 23, 24, 25, 26, 27,

28, 29, 30, 31, 32, 33, 34, 35, 36, 37, 38, 39 or 40.

Preferably the Pel-ase glycoside hydrolase is an isolated glycoside hydrolase.

Preferably the Pel-ase glycoside hydrolase enzyme is present in the machine-cleaning composition in an amount from 0.001 to 1 wt% based on active protein in the composition, or from 0.005 to 0.5 wt% or from 0.01 to 0.25 wt% based on weight of the composition.

Preferably the glycoside hydrolase enzyme is present in the aqueous wash liquor in an amount of from O.Olppm to 1000 ppm enzyme, based on active protein or from 0.05 or from 0.1ppm to 750 or 500ppm.

The Pel-ase described herein may also give rise to biofilm-disrupting effects or soil anti-redeposition effects.

Psl-ase enzyme

The Psl-ase enzyme exhibits glycoside hydrolase activity towards Psl polysaccharide. The Pel-ase preferably has at least 60% or 65% or more preferably at least 70% or 75% or 80% or 85% or 90% or 95% up to 100% identity to one or more of SEQ ID NO: 41, 42, 43, 44, 45, 46, 47, 48, 49, 50, 51 or 52.

Preferably the glycoside hydrolase is an isolated glycoside hydrolase.

Preferably the Psl-ase glycoside hydrolase enzyme is present in the cleaning composition in 20 an amount from 0.001 to 1 wt%, or from 0.005 to 0.5 wt% or from 0.01 to 0.25 wt% based on active protein.

Preferably the Psl-ase glycoside hydrolase enzyme is present in a laundering aqueous liquor in an amount of from O.Olppm to 1000 ppm enzyme, based on active protein or from 0.05 or from 0.1ppm to 750 or 500ppm.

The composition comprising the Psl-ase glycoside hydrolase described herein may also give rise to/be useful for biofilm-disrupting effects or soil anti-redeposition effects.

Endo-beta-1,3(4)-glucanase Enzyme

The endo-beta-1,3(4)-glucanase enzyme exhibits significant endo-beta-1,3-glucanase activity against polysaccharides with an endo-beta-1,3-glucan backbone (e.g. curdlan, pachyman, laminarin, scleroglucan, schizophyllan) and so-called 'mixed linkage' glucan polysaccharides that contain both beta-1,3- and beta-1,4 linkages such as those found in cereals such as oats and barley. Activity against both of these substrates can be confirmed using Test Method 2. The endo-beta-1,3(4)-glucanase enzyme preferably belongs to the class E.C. 3.2.1.6. The endo-beta-1,3(4)-glucanase preferably has at least 60% or 65% or more preferably at least 70% or at least 75% or at least 80% or at least 85% or at least 90% or at least 95% up to 100% identity to one or more of SEQ ID NOs: 53, 54, 55, 56, 57 or 58.

Endo-beta-1,3-glucanase Enzyme

The endo-beta-1,3-glucanase enzyme exhibits significant endo-beta-1,3-glucanase activity against polysaccharides with an endo-beta-1,3-glucan backbone (e.g. curdlan, pachyman, laminarin, scleroglucan, schizophyllan) but has little or no activity against so-called 'mixed linkage' glucan polysaccharides that contain both beta-1,3- and beta-1,4 linkages such as those found in cereals such as oats and barley. Activity against both of these substrates can be determined using Test Method 2. The endo-beta-1,3-glucanase enzyme preferably belongs to the class E.C. 3.2.1.39. The endo-beta-1,3-glucanase preferably has at least 60% or 65% or more preferably at least 70% or at least 75% or at least 80% or at least 85% or at least 90% or at least 95% up to 100% identity to one or more of SEQ ID NOs: 59, 60, 61, 62, 63, 64 or 65.

The machine-cleaning composition of the invention may comprise at least alginate lyase enzyme and Pel-ase enzyme, and optionally additionally, Psl-ase enzyme, endo-β-1,3-glucanase enzyme and/or endo-β-1,3(4)- glucanase enzyme.

The machine-cleaning composition of the invention may comprise at least alginate lyse enzyme and Psl-ase enzyme, and optionally additionally, Pel-ase enzyme, endo-β-1,3-glucanase enzyme and/or endo-β-1,3(4)-glucanase enzyme.

The machine-cleaning composition of the invention may comprise at least alginate lyase enzyme and endo-β-1,3-glucanase enzyme, and optionally additionally, Pel-ase enzyme, Psl-ase enzyme and/or endo-β-1,3(4)-glucanase enzyme.

The machine-cleaning composition of the invention may comprise at least alginate lyase enzyme and endo-β-1,3(4)-glucanase enzyme, and optionally additionally, Pel-ase enzyme, Psl-ase enzyme, and/or endo-β-1,3-glucanase enzyme.

The machine-cleaning composition of the invention may comprise at least Pel-ase enzyme and Psl-ase enzyme, and optionally additionally, alginate lyase enzyme, endo-β-1,3-glucanase enzyme and/or endo-β-1,3(4)-glucanase enzyme.

The machine-cleaning composition of the invention may comprise at least Pel-ase enzyme and endo-β-1,3-glucanase enzyme, and optionally additionally, alginate lyase enzyme, Psl-ase enzyme, and/or endo-β-1,3(4)- glucanase enzyme.

The machine-cleaning composition of the invention may comprise at least Pel-ase enzyme and endo-β-1,3(4)glucanase enzyme, and optionally additionally, Psl-ase enzyme, endo-β-1,3-glucanase enzyme and/or alginate lyase enzyme.

The machine-cleaning composition of the invention may comprise at least Psl-ase enzyme and endo-β-1,3-glucanase enzyme and optionally additionally, Pel-ase enzyme, alginate lyase enzyme and/or endo-β-1,3(4)-glucanase enzyme.

The machine-cleaning composition of the invention may comprise at least Psl-ase enzyme and endo-β-1,3(4)-glucanase enzyme, and optionally additionally, Pel-ase enzyme, endo-β-1,3-glucanase enzyme and/or alginate lyase enzyme.

The machine-cleaning composition of the invention may comprise at least endo-β-1,3-glucanase enzyme and endo-β-1,3(4)- glucanase enzyme, and optionally additionally, Pel-ase enzyme, Psl-ase enzyme, and/or alginate lyase enzyme.

The machine-cleaning composition comprises a cleaning adjunct. Typically the cleaning adjunct will be present in the composition in an amount from 1 to 98.9 wt%, more typically from 5 to 90 wt% cleaning adjunct. Suitable cleaning adjuncts comprise: surfactants, builders, bleach ingredients, colorants, chelating agents, dispersants, additional enzymes, and enzyme stabilizers, catalytic materials, preformed peracids, polymeric dispersing agents, clay soil removal/anti-redeposition agents, filler salts, hydrotropes, suds suppressors, structure elasticizing agents, preservatives, anti-oxidants, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; flocculants such as polyethylene oxide; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of dicarboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof. The composition may be such that the cleaning adjunct preferably comprises one or more selected from the group consisting of (i) surfactant; (ii) additional enzyme; (iii) suds supressor, (iv) enzyme stabilizer; (v) solvent; and (vi) mixtures thereof. The composition may also contain bleach ingredients.

The present inventors have found that the enzyme combination provides good soil breakdown, however the removal of the products of the breakdown of the substrates and soils containing may be improved by the presence of surfactant. The machine-cleaning composition therefore preferably comprises a surfactant, which may be selected from anionic, nonionic, zwitterionic, amphoteric and cationic surfactants, preferably selected from anionic surfactant, nonionic surfactant and mixtures thereof, preferably in an amount from 1 to 60 wt%. Nonionic surfactants are particularly preferred. The composition may be free of anionic surfactant.

Preferably the weight ratio of surfactant to either total active enzyme protein, or active enzyme protein of each of the at least two enzymes is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1. Preferably the weight ratio of surfactant to active enzyme protein is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1.

Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

Where present, the anionic surfactant is preferably added to the machine-cleaning composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or tri-ethanolamine. The composition may comprise from 1 to 60 weight % or from 1 to 50 wt% or 2 or 5 to 40 wt% of the composition, anionic surfactant. The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The invention also provides a machine-cleaning composition comprising: from 0.00005 to 5 wt% (active enzyme protein) of each of the at least two enzymes and a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ™, Lutensol™ and Pluronic™ from BASF, Dehypon™ series from Cognis and Genapol™ series from Clariant.

The machine-cleaning composition preferably comprises from 0.5wt% to about 40wt% of a non-ionic surfactant, preferably 1 to 30 wt% of the composition non-ionic surfactant.

The machine-cleaning composition preferably comprises one or more additional enzymes. Therefore a preferred composition comprises one or more additional enzymes selected from the group consisting of aminopeptidase, amylase, carbohydrase, carboxypeptidase, catalase, cellulase, chitinase, cutinase, cyclodextrin glycosyltransferase, esterase, alpha-galactosidase, beta-galactosidase, glucoamylase, alpha-glucosidase, beta-glucosidase, haloperoxidase, hexosaminidase, invertase, laccase, lipase, mannanase, mannosidase, nuclease, oxidase, pectinolytic enzyme, peptidoglutaminase, peroxidase, phytase, polyphenoloxidase, proteolytic enzyme, transglutaminase, xylanase, xanthan lyase, xanthanase, and mixtures thereof. Preferably the cleaning composition comprises additional enzyme selected from nuclease, such as DNase or RNase, mannanase, xanthan lyase, xanthanase, amylase and mixtures thereof.

Preferably the composition comprises additional enzymes selected from xanthan lyase, xanthanase, mannanase and mixtures thereof. Mannanase is particularly preferred.

The additional enzyme(s) may be produced, for example, by a microorganism belonging to the genus *Aspergillus, e.g., Aspergillus aculeatus*, *Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis*, *Fusarium crookwellense, Fusarium culmorum, Fusarium graminearum, Fusarium graminum, Fusarium heterosporum, Fusarium negundi, Fusarium oxysporum, Fusarium reticulatum*, *Fusarium roseum, Fusarium sambucinum, Fusarium sarcochroum, Fusarium sulphureum, Fusarium toruloseum, Fusarium trichothecioides,* or *Fusarium venenatum*; *Humicola*, *e.g., Humicola insolens* or *Humicola lanuginosa;* or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease, a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Preferably, the product of the invention comprises at least 0.01 mg, preferably from about 0.05 to about 10, more preferably from about 0.1 to about 6, especially from about 0.2 to about 5 mg of active further enzyme/ g of composition.

Proteases: The composition of the invention preferably comprises a protease. A mixture of two or more proteases

can contribute to an enhanced cleaning across a broader temperature, cycle duration, and/or substrate range. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

subtilisins (EC 3.4.21.62), especially those derived from *Bacillus,* such as *Bacillus sp., B. lentus, B. alkalophilus*, *B. subtilis, B. amyloliquefaciens, B. pumilus, B. gibsonii,* and *B. akibaii* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569 and WO2016174234. Specifically, mutations S9R, A15T, V66A, A188P, V199I, Q239R, N255D (savinase numbering system).

trypsin-type or chymotrypsin-type proteases, such as trypsin (e.g., of porcine or bovine origin), including the Fusarium protease described in WO 89/06270 and the chymotrypsin proteases derived from *Cellumonas* described in WO 05/052161 and WO 05/052146.

metalloproteases, especially those derived from *Bacillus amyloliquefaciens* decribed in WO07/044993A2; from *Bacillus, Brevibacillus, Thermoactinomyces*, *Geobacillus, Paenibacillus, Lysinibacillus* or *Streptomyces spp.* described in WO2014194032, WO2014194054 and WO2014194117; from *Kribella alluminosa* described in WO2015193488; and from *Streptomyces* and *Lysobacter* described in WO2016075078.

protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Especially preferred proteases for the cleaning composition of the invention are polypeptides having at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).

(i) G118V + S128L + P129Q + S130A

(ii) S101M + G118V + S128L + P129Q + S130A

(iii) N76D + N87R + G118R + S128L + P129Q + S130A + S188D + N248R

(iv) N76D + N87R + G118R + S128L + P129Q + S130A + S188D + V244R

(v) N76D + N87R + G118R + S128L + P129Q + S130A

(vi) V68A + N87S + S101G + V104N

(vii) S99AD

(viii) S9R+A15T+V68A+N218D+Q245R

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase®, Coronase®, Blaze®, Blaze Ultra® and Esperase® by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/Kemira, namely BLAP (sequence shown in Figure29 of US 5,352,604

with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (Bacillus alkalophilus subtilisin with mutations A230V + S256G + S259N) from Kao.

Especially preferred for use herein are commercial proteases selected from the group consisting of Properase®, Blaze®, Ultimase®, Everlase®, Savinase®, Excellase®, Blaze Ultra®, BLAP and BLAP variants.

Preferred levels of protease in the product of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces*), e.g., from *H. lanuginosa* (*T. lanuginosus*) or from *H. insolens,* a *Pseudomonas lipase,* e.g., from *P. alcaligenes* or *P. pseudoalcaligenes*, *P. cepacia P. stutzeri, P. fluorescens, Pseudomonas* sp. strain SD 705, *P. wisconsinensis* , a *Bacillus* lipase, e.g., from *B. subtilis* (Dartois et al. (1993), Biochemica et Biophysica Acta, 1131, 253-360), *B. stearothermophilus* or *B. pumilus* .

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot 059952 (derived from *Thermomyces lanuginosus (Humicola lanuginosa)).* Preferred lipases include those sold under the tradenames Lipex®, Lipolex® and Lipoclean®.

Other suitable lipases include: Liprl 139, e.g. as described in WO2013/171241; TfuLip2, e.g. as described in WO2011/084412 and WO2013/033318; Pseudomonas stutzeri lipase, e.g. as described in WO2018228880; *Microbulbifer thermotolerans* lipase, e.g. as described in WO2018228881; *Sulfobacillus acidocaldarius* lipase, e.g. as described in EP3299457; LIP062 lipase e.g. as described in WO2018209026; PinLip lipase e.g. as described in WO2017036901 and Absidia sp. lipase e.g. as described in WO2017005798.

A suitable lipase is a variant of SEQ ID NO:5 comprising:

(a) substitution T231R
and
(b) substitution N233R or N233C
and
(c) at least three further substitutions selected from E1C, D27R, N33Q, G38A, F51V, G91Q, D96E, K98L, K98I, D111A, G163K, H198S, E210Q, Y220F, D254S, I255A, and P256T;

where the positions correspond to the positions of SEQ ID NO:5 and wherein the lipase variant has at least 90% but less than 100% sequence identity to the polypeptide having the amino acid sequence of SEQ ID NO: 5 and wherein the variant has lipase activity.

One preferred lipase is a variant of SEQ ID NO: 5 comprising the following substitutions: T231R, N233R, D27R, G38A, D96E, D111A, G163K, D254S and P256T

One preferred lipase is a variant of SEQ ID NO: 5 comprising the following substitutions: T231R, N233R, N33Q, G91Q, E210Q, I255A.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307 , US 5,648,263 , US 5,691,178, US 5,776,757 and US 5,691,178 .

In one aspect, preferred enzymes include microbial-derived endoglucanases exhibiting endo-beta-1,4-glucanase activity (E.C. 3.2.1.4), preferably selected from the group comprising:

(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 246, 237, 224 and 221 of the mature polypeptide of SEQ ID NO: 2, and t he variant has cellulase activity.;
(b) a glycosyl hydrolase having enzymatic activity towards both xyloglucan and amorphous cellulose substrates,

wherein the glycosyl hydrolase is selected from GH families 5, 7, 12, 16, 44 or 74;

(c) a glycosyl hydrolase having a sequence of at least 90%, 94%, 97% and even 99% identity to the amino acid sequence SEQ ID NO:3 in WO09/148983;

(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;

(e) and mixtures thereof.

Suitable endoglucanases are sold under the tradenames Celluclean® and Whitezyme® (Novozymes A/S, Bagsvaerd, Denmark). Examples include Celluclean® 5000L, Celluclean® Classic 400L, Celluclean® Classic 700T, Celluclean® 4500T, Whitezyme® 1.5T, Whitezyme® 2.0L.

Other commercially available cellulases include Celluzyme®, Carezyme®, Carezyme® Premium (Novozymes A/S), Clazinase®, Puradax HA®, Revitalenz® 1000, Revitalenz® 2000 (Genencor International Inc.), KAC-500(B)® (Kao Corporation), Biotouch® FCL, Biotouch® DCL, Biotouch® DCC, Biotouch® NCD, Biotouch® FCC, Biotouch® FLX1 (AB Enzymes)

Suitable glucanases include endo-β-1,3-glucanases, preferably from E.C. class 3.2.1.39, preferably obtained from *Paenibacillus sp, Zobellia galactanivorans*, *Thermotoga petrophila* or *Trichoderma sp* micro-organism, preferably *Paenibacillus sp* or *Zobellia galactanivorans*, most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.

(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.

(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, 1206, M208, D209, E212, V213, V214, N214, L217, R218, N219, V222, T225, T227, G229, 1235, K242, Y243, S244, F245, T246, 1250, S255, A256, H286, V291, T316, V317, V318, N417, T418, A419, H420, P421, 1428, M429, F440, R443, N444, K445, Q448, S451, A465, N470, S472.

(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.

(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.

(f) variants exhibiting at least 89% identity with SEQ ID NO:1 in WO2016091688, especially those comprising deletions at positions H183+G184 and additionally one or more mutations at positions 405, 421, 422 and/or 428.

(g) variants described in WO2014099523, especially those exhibiting at least 60% amino acid sequence identity with the "PcuAmyl α-amylase" from *Paenibacillus curdlanolyticus* YK9 (SEQ ID NO:3 in WO2014099523).

(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178*, G179*, T38N, N88H, N126Y, T129I, N134M, F153W, L171R, T180D, E187P, I203Y, G476K, G477E, Y303D.

(i) variants exhibiting at least 85% identity with AmyE from *Bacillus subtilis* (SEQ ID NO:1 in WO2009149271).

(j) variants exhibiting at least 90% identity with the wild-type amylase from Bacillus sp. KSM-K38 with accession number AB051102.

(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H*, N54S, V56T, K72R, G109A, F113Q, R116Q, W167F, Q172G, A174S, G184T, N195F, V206L, K391A, P473R, G476K.

(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL®, ATLANTIC®, ACHIEVE ALPHA®, AMPLIFY® PRIME, INTENTSA® and BAN® (Novozymes A/S, Bagsvaerd, Denmark), KEMZYM® AT 9000 Biozym Biotech Trading GmbH Wehlistrasse 27b A-1200 Wien Austria, RAPIDASE® , PURASTAR®, ENZYSIZE®, OPTISIZE HT PLUS®, POWERASE®, PREFERENZ S® series (including PREFERENZ S1000® and PREFERENZ S2000® and PURASTAR OXAM® (DuPont., Palo Alto, California) and KAM® (Kao, 14-10 Nihonbashi Kayabacho, 1-chome, Chuoku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from *C. cinereus,* and variants thereof as those described in WO 93/24618, WO 95/10602, and WO 98/15257.

Commercially available peroxidases include GUARDZYME® (Novozymes A/S).

Pectate lyase: Suitable pectate lyases include those sold under the tradenames Pectawash®, Pectaway®, X-Pect®, (all Novozymes A/S, Bagsvaerd, Denmark) Preferenz® F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway® (Novozymes A/S) such as Mannaway® 200L and Mannaway Evity 4.0T Other commercially available mannanases include Effectenz® M1000, Mannastar® 375, Preferenz M100 and Purabrite® (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Nucleases: Preferably the composition comprises a nuclease enzyme such as a RNase or DNase or mixtures thereof. The nuclease enzyme is an enzyme capable of cleaving the phosphodiester bonds between the nucleotide subunits of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus, it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1

and mixtures thereof.

DNase: Suitable DNases include wild-types and variants of DNases defined by SEQ ID NOS: 1, 2, 3, 4, 5, 6, 7, 8 and 9 in WO2017162836 (Novozymes), and SEQ ID NOS: 1, 2, 3, 4, 5, 6, 7, 8, 9, 10, 11, 12, 13, 14, 15, 16, 17, 18, 19, 20, 21, 22, 23, 24, 25, 28, 29, 30, 31, 32, 33, 34, 35, 36, 37, 38 39, 40, 41, 42, 43, 44, 45, 46, 47, 48, 49 50, 51, 52, 53, and 54 in WO2018108865, and variants of the Bacillus cibi DNase including those described in WO2018011277 (Novozymes), incorporated herein by reference. Preferred DNases are as claimed in EP3476935A.

RNase: suitable RNases include wild-types and variants of DNases defined by SEQ ID NOS: 3, 6, 9, 12, 15, 57, 58, 59, 60, 61, 62, 63, 64, 65, 66, 67, 72 and 73 in WO2018178061 (Novozymes), and SEQ ID NOS: 86, 87, 88, 89, 90, 91, 92, 93, 94, 95, 96, 97, 98, 99, 100, 101, 102, 103 and 104 in WO2020074499 (Novozymes), incorporated herein by reference.

Hexosaminidase: The composition preferably additionally comprises one or more hexosaminidase enzymes. The term hexosaminidase includes "dispersin" and the abbreviation "Dsp", which means a polypeptide having hexosaminidase activity. Suitable enzymes are found in EC 3.2.1.- that catalyze the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 are preferred, especially the variants with improved thermostability disclosed in that publication.

Particularly preferred hexosaminidase enzymes are hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 66; and hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 67 herein; and mixtures thereof.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

Galactanase: Preferably the composition comprises a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the machine-cleaning composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Builders: The machine-cleaning composition may further contain builders, such as builders based on carbonate, bicarbonate or silicates which may be Zeolites, such as Zeolite A, Zeolite MAP (Maximum Aluminium type P). Zeolites, useable in laundry preferably has the formula $Na12(AlO2)12(SiO2)12\cdot 27H2O$ and the particle size is usually between 1-10 μm for zeolite A and 0.7-2 um for zeolite MAP. Other builders are Sodium metasilicate ($Na2SiO3 \cdot nH2O$ or $Na2Si2O5 \cdot n H2O$) strong alkaline and preferably used in dish wash. In preferred embodiments, the amount of a

detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; enzymes; anti-bacterial agents; bleaches; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof.

Enzyme stabilizer: The composition preferably comprises an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: The composition may comprise a structurant for example selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition may comprise one or more polymers. Preferred examples are carboxymethylcellulose, poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof. Amphiphilic cleaning polymers: Preferably, the amphiphilic cleanimg polymer is a compound having the following general structure: bis($(C_2H_5O)(C_2H_4O)n$)($CH_3$)-$N^+$-$C_xH_{2x}$-$N^+$-($CH_3$)-bis($(C_2H_5O)(C_2H_4O)n$), wherein n = from 20 to 30, and x = from 3 to 8, or sulphated or sulphonated variants thereof.

Amphiphilic alkoxylated grease cleaning polymers refers to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV):

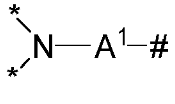

(I)

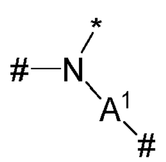

(II)

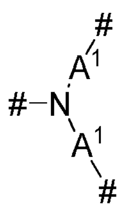

(III)

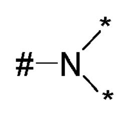

(IV)

wherein # in each case denotes one-half of a bond between a nitrogen atom and the free binding position of a group $A^1$ of two adjacent repeating units of formulae (I), (II), (III) or (IV); * in each case denotes one-half of a bond to one of the alkoxylate groups; and $A^1$ is independently selected from linear or branched $C_2$-$C_6$-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b),

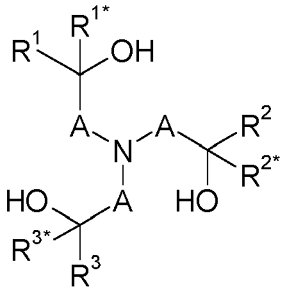

(I.a)

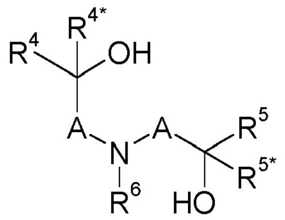

(I.b)

wherein A are independently selected from $C_1$-$C_6$-alkylene; $R^1$, $R^{1*}$, $R^2$, $R^{2*}$, $R^3$, $R^{3*}$, $R^4$, $R^{4*}$, $R^5$ and $R^{5*}$ are independently selected from hydrogen, alkyl, cycloalkyl or aryl, wherein the last three mentioned radicals may be optionally substituted; and $R^6$ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V)

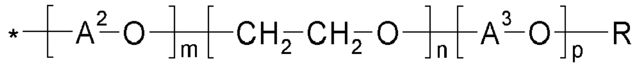

(V)

wherein * in each case denotes one-half of a bond to the nitrogen atom of the repeating unit of formula (I), (II) or (IV); $A^2$ is in each case independently selected from 1,2-propylene, 1,2-butylene and 1,2-isobutylene; $A^3$ is 1,2-propylene; R is in each case independently selected from hydrogen and $C_1$-$C_4$-alkyl; m has an average value in the range of from 0 to about 2; n has an average value in the range of from about 20 to about 50; and p has an average value in the range of from about 10 to about 50.

Carboxylate polymer: The composition may also include one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):

(I) $-[(OCHR^1-CHR^2)_a-O-OC-Ar-CO-]_d$

(II) $-[(OCHR^3-CHR^4)_b-O-OC-sAr-CO-]_e$

(III) $-[(OCHR^5-CHR^6)_c-OR^7]_f$

wherein:

a, b and c are from 1 to 200;
d, e and f are from 1 to 50;
Ar is a 1,4-substituted phenylene;
sAr is 1,3-substituted phenylene substituted in position 5 with $SO_3Me$;
Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
$R^1$, $R^2$, $R^3$, $R^4$, $R^5$ and $R^6$ are independently selected from H or $C_1$-$C_{18}$ n- or iso-alkyl; and
$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including

Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

## Method of Use

The present invention also provides a method of treating the interior of a washing machine, such as a dishwashing machine or laundry washing machine to prevent or remove the build-up of soils, the method comprising the step of supplying the machine-cleaning composition to the interior of the machine during at least one wash cycle or at least one rinse cycle of the dishwashing machine or laundry washing machine the machine-cleaning composition being present in the wash or rinse water (aqueous wash liquor).

In particular, the method is for treating the interior of a laundry washing machine.

The invention also provides a method of degrading soils on an interior surface of a washing machine such as a dishwashing machine or laundry washing machine, which method comprises the steps of providing the machine-cleaning composition during at least one or more wash cycles or one or more rinse cycles of the washing machine, the machine-cleaning composition being present in the wash or rinse water (aqueous wash liquor).

The invention also provides use of a machine-cleaning composition for cleaning a washing machine such as a laundry washing machine or dishwashing machine. The compositions and methods of the invention are suitable for cleaning either domestic or commercial/institutional washing machines.

The machine-cleaning composition may be provided to the washing machine either via a dosing mechanism which is integral to the machine, such as the drawer or other dispenser, or via a cartridge which doses into the machine, or by being placed directly into the inside of the machine, for example in the case of a laundry washing machine, by being placed directly into the drum.

The washing machine may be at least partially loaded with articles during the at least one wash cycle or at least one rinse cycle. The machine-cleaning composition may be used over two or more sequential wash cycles or rinse cycles of the washing machine.

It may be preferred for the machine to be empty during the at least one wash cycle or at least one rinse cycle, so that the machine-cleaning composition of the invention has better access to all areas of the washing machine. This is particularly the case for a laundry washing machine, thus in a preferred method for cleaning a laundry washing machine, the at least one wash cycle or at least one rinse cycle is carried out in an empty washing machine.

In a preferred embodiment of the invention, the machine-cleaning composition is used in two or more operations of the washing machine, for example this may be in the same wash programme for example in two or more of a pre-wash, wash and rinse step, or it may be in sequential wash programmes. It is preferred that these operations are sequential operations. The cleaning operation may comprise a pre-wash (optional), a wash cycle and a rinse cycle or

may comprise either a wash-only cycle or a rinse-only cycle. This rinse-only cycle may be either a pre-wash cycle or a post-wash cycle. The use of the compositions of the invention in only a wash cycle, or, only a rinse cycle may be achieved by either dosing the composition into the washing machine which is then run on only that type of cycle with the composition present. Alternatively, the compositions may be formulated with a suitable release mechanism e.g. accelerated or delayed release so that the cleaning composition is released at the desired part of the washing operation. Where a polymer is to be used to achieve an accelerated or delayed release of the composition of the invention, suitable polymers to achieve this effect are well known in the art.

The washing machine may be run on any of its programmes during the method including both wash and/or rinse programmes. Typically a wash and rinse cycle may last up to 2 hours, with a wash-only cycle lasting up to 1.5 hours and a rinse-only cycle lasting up to 0.5 hours.

It is preferred that the composition of the invention is in contact with the soil to be removed for a period of at least 10 minutes, preferably at least 15 minutes, such as at least 20 minutes regardless of whether the composition is applied to the soils during the wash or rinse cycle.

During the wash/rinse cycle(s) the composition is distributed throughout the cavity of the washing machine. This provides contact with the surfaces of the interior of the machine e.g. the pipes, filter drum etc. Additionally, the aqueous wash liquor containing the composition of the invention must drain through the filter and pipework thereby providing additional opportunities for removal of soils therefrom.

In the aqueous wash liquor the or each enzyme is preferably present in an amount from O.Olppm to 10ppm, preferably from 0.1ppm to 1ppm. Where present, surfactant is preferably present in an amount from 0.05 to 5g/l, or up to 2g/l, or up to 1g/l or up to 0.75g/l or up to 0.5g/l.

The or each enzyme may be present in the wash liquor in an amount corresponding to 0.001-100 mg of active enzyme protein per liter of wash liquor, preferably 0.005-5 mg of active enzyme protein per liter of wash liquor, more preferably 0.01-1 mg of enzyme protein per liter of wash liquor and in particular 0.1-1 mg of enzyme protein per liter of wash.

The wash liquor preferably has a pH of from about 5 or 10.5, more preferably from 6 to 8, or 7.5 The wash liquor preferably has a temperature from about 5 °C to about 40 °C, or preferably from 10 to 35 °C or 30 °C or 25 °C.

TEST METHODS

Test Method 1

Enzymatic activity towards β-D-mannuronic acid blocks (polyM activity) and α-L-guluronic acid blocks (polyG activity)

The alginate lyase activity is measured using Mannuronic Block Oligosaccharide DP20-DP35 (Product code: ALG601) and Guluronate oligosaccharide DP2-DP45 (Product code: ALG610) from Elicityl, France as substrates. Mannuronic Block Oligosaccharide DP20-DP35 is used to measure polyM activity, whereas Guluronate oligosaccharide DP2-DP45 was used for measuring polyG activity.

A solution of 2.5% of each substrate was suspended in Tris buffer at pH 8.3 and incubated with 3 ppm of each alginate lyase of interest, at 25°C for 60 min, in a 96 well plate.

The activity towards each of the substrates is given as the delta absorbance at 235nm in a spectrophotometer vs nil enzyme sample when the enzyme was put in contact with each of the substrates. These values are then used to evaluate the activity towards β-D-mannuronic acid blocks (polyM) and/or α-L-guluronic acid blocks (polyG) of the respective enzymes. An enzyme having activity towards poly(beta-D-mannuronate) (polyM activity) preferably provides a delta absorbance versus nil enzyme of at least 0.1 absorbance units, more preferably at least 0.15 absorbance units and more preferably at least 0.2 absorbance units. An enzyme having activity towards poly(alpha-L-guluronate) (polyG activity) preferably provides a delta absorbance versus nil enzyme of at least 0.3 absorbance units, preferably at least 0.4 or even 0.5 or 0.6 absorbance units.

Test Method 2

Measurement of activity against beta-1,3-glucan and beta-1,3(4)-glucan

Activity against beta-1,3-glucan and beta-1,3(4)-glucan was measured using the red variant of the Discovery kit supplied by GlycoSpot A/S, Søborg, Denmark which allows simultaneous assay of a given enzyme across 16 different activities. Protocol for the assay is given below: if a given enzyme shows greater than 0.3 absorbance units against the curdlan (CPH0009) AND beta glucan from barley (CPH0003) it is deemed to be active towards both beta-1,3-glucan and beta-1,3(4)-glucan, i.e. be classified as an endo-beta-1,3(4)-glucanase If the enzyme shows greater than 0.3 absorbance units against the curdlan (CPH0009) but less than or equal to 0.3 absorbance units against the beta glucan from barley (CPH0003) it is deemed to be an endo-beta-1,3-glucanase.

1. Add 200 μl activation solution to each well in the 96-well plate.
2. Incubate 10 minutes at room temperature without agitation.
3. Remove the remaining solution by centrifugation (2700 xg for 10 min or vacuum).
4. Wash twice with 100 μl water to remove the stabiliser completely.
5. Add samples and controls to each well in the 96-well plate: The controls are 150 μl 1M tris(hydroxymethyl)aminomethane hydrochloride (Tris-HCL) buffer (pH 8). The samples are 1mg of active enzyme protein dissolved in 150 μl of the same Tris-HCL buffer.
6. Put a 'product plate' underneath the substrate plate.
7. Incubate the reaction at 30°C for 30 minutes.
8. Transfer the reaction product into the product plate by centrifugation (2700 xg for 10 min or vacuum).
9. Check that the volume in each well is equal.
10. Detect the absorbance (517 nm).

EXAMPLES

MACHINE CLEANING COMPOSITION EXAMPLES

Examples 1-3 Machine Cleaning Gels:

| | 1 (wt %) | 2 (wt %) | 3 (wt %) |
|---|---|---|---|
| Tri sodium citrate | 22.0 | 15.0 | 23.4 |
| Acrylic copolymer | 5.0 | 2.3 | 3.3 |
| Disodium citrate | 2.6 | 4.3 | 4.2 |
| Benzalkonium chloride | 0.9 | 3.1 | 0.0 |
| Perfume | 0.2 | 0.4 | 0.2 |
| C12-15 Alcohol ethoxylate(11 EO) nonionic surfactant | 1.1 | 2.1 | 3.1 |
| Xanthan gum | 0.4 | 0.3 | 0.2 |
| DNase as defined herein (mg active per 100g composition) | 5.0 | 3.4 | 3.3 |
| Alginate lyase as defined herein (mg active per 100g of detergent) | 5.3 | 5.8 | 4.2 |
| Hexosaminidase as defined herein (mg active per 100g composition) | 3.1 | 2.3 | 2.1 |
| Pel-ase as defined herein (mg active per 100g composition) | 3.3 | 9.2 | 0.0 |
| Psl-ase as defined herein (mg active per 100g composition) | 7.2 | 0.0 | 3.2 |
| Endo-beta-1,3(4)-glucanase as defined herein (mg active per 100g composition) | 3.2 | 3.6 | 4.3 |
| Endo-beta-1,3-glucanase as defined herein (mg active per 100g composition) | 1.5 | 8.3 | 0.0 |
| Water | Balance | Balance | Balance |

Examples 4-6 Machine Cleaning Powders

| | 4 (wt %) | 5 (wt %) | 6 (wt %) |
|---|---|---|---|
| Zeolite | 45.0 | 33.1 | 44.2 |
| Water | 4.3 | 5.3 | 4.5 |
| Sodium bentonite | 7.0 | 4.3 | 2.3 |
| C12-14 Alcohol ethoxylate(5 EO) nonionic surfactant | 2.1 | 1.1 | 0.3 |

(continued)

| | 4 (wt %) | 5 (wt %) | 6 (wt %) |
|---|---|---|---|
| Sodium polycarboxylate (maleate/acrylate copolymer) | 2.2 | 5.3 | 3.4 |
| DNase as defined herein (mg active per 100g composition) | 5.3 | 3.7 | 1.9 |
| Alginate lyase as defined herein (mg active per 100g of detergent) | 3.3 | 2.8 | 3.2 |
| Hexosaminidase as defined herein (mg active per 100g composition) | 3.4 | 2.7 | 2.4 |
| Pel-ase as defined herein (mg active per 100g composition) | 3.3 | 6.2 | 0.0 |
| Psl-ase as defined herein (mg active per 100g composition) | 3.2 | 0.0 | 2.2 |
| Endo-beta-1,3(4)-glucanase as defined herein (mg active per 100g composition) | 3.8 | 3.8 | 4.0 |
| Endo-beta-1,3-glucanase as defined herein (mg active per 100g composition) | 1.2 | 1.3 | 0.0 |
| Sodium sulfate | Balance | Balance | Balance |

Examples 7-9 Machine Cleaning Tablets

| | 7 (wt %) | 8 (wt %) | 9 (wt %) |
|---|---|---|---|
| Sodium citrate | 43.3 | 23.4 | 33.5 |
| Sodium polyacrylate | 3.4 | 7.3 | 3.0 |
| Microcrystalline cellulose | 2.3 | 1.9 | 2.2 |
| PEG-150 | 1.1 | 0.7 | 1.2 |
| Sodium bicarbonate | 8.2 | 4.2 | 2.0 |
| Talc | 0.8 | 1.0 | 2.3 |
| DNase as defined herein (mg active per 100g composition) | 5.2 | 3.6 | 3.9 |
| Alginate lyase as defined herein (mg active per 100g of detergent) | 2.3 | 4.8 | 3.4 |
| Hexosaminidase as defined herein (mg active per 100g composition) | 3.4 | 3.7 | 2.4 |
| Pel-ase as defined herein (mg active per 100g composition) | 6.3 | 3.2 | 0.0 |
| Psl-ase as defined herein (mg active per 100g composition) | 7.2 | 0.0 | 2.1 |
| Endo-beta-1,3(4)-glucanase as defined herein (mg active per 100g composition) | 3.7 | 4.8 | 3.0 |
| Endo-beta-1,3-glucanase as defined herein (mg active per 100g composition) | 5.5 | 1.2 | 0.0 |
| Sodium sulfate | Balance | Balance | Balance |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A washing machine-cleaning composition comprising (a) at least two enzymes selected from (i) alginate lyase enzyme; (ii) Pel-ase enzyme; (iii) Psl-ase enzyme; (iv) endo-β-1,3-glucanase enzyme; and (v) endo-β-1,3(4)- glucanase enzyme; and (b) a cleaning adjunct.

2. A washing machine-cleaning composition according to claim 1 additionally comprising a surfactant.

3. A washing machine-cleaning composition according to claim 1 or claim 2 wherein the alginate lyase enzyme is of microbial origin, preferably bacterial or algal, most preferably bacterial, preferably obtainable from *Flavobacterium sp, Sphingomonas sp*, *Zobellia galactanivorans*, *preferably Flavobacterium sp.*

4. A washing machine-cleaning composition according to any preceding claim wherein the alginate lyase enzyme comprises: an alginate lyase selected from alginate lyase having at least 60%, or at least 70%, or at least 80%, or at least 90%, or at least 95%, or at least 96%, or at least 97%, or at least 98%, or at least 99%, or 100%, sequence identity to one or more of SEQ ID NO: 1; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 3; an alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 7, and mixtures thereof, preferably being selected from alginate lyase having at least 60% sequence identity to one or more of SEQ ID NO: 1; SEQ ID NO: 5; SEQ ID NO: 6; and SEQ ID NO: 7, or mixtures thereof, most preferably wherein the alginate lyase enzyme comprises: an alginate lyase selected from alginate lyase having at least 60%, sequence identity to one or more of SEQ ID NO: 6; and SEQ ID NO: 7 and mixtures thereof.

5. A washing machine-cleaning composition according to any preceding claim additionally comprising a nuclease enzyme and/or hexosaminidase enzyme in an amount from 0.00005 to 5 wt% active enzyme protein.

6. A washing machine-cleaning composition according to any preceding claim wherein the composition additionally comprises nonionic surfactant, preferably in an amount from 0.5 to 30 wt% of the composition.

7. A washing machine-cleaning composition according to any preceding claim comprising additional enzyme, preferably selected from amylase, nuclease, mannanase, xanthan lyase, xanthanase, and mixtures thereof.

8. A method of treating the interior of a washing machine, such as a dishwashing machine or laundry washing machine to prevent or remove the build-up of soils, the method comprising the step of supplying the machine-cleaning composition according to any preceding claim to the interior of the machine during at least one wash cycle or at least one rinse cycle of the dishwashing machine or laundry washing machine.

9. A method according to claim 8 for treating the interior of a laundry washing machine.

10. A method of degrading soils on an interior surface of a dishwashing machine or laundry washing machine, which method comprises the steps of providing the machine-cleaning composition according to any of claims 1 to 7 during at least one or more wash cycles or one or more rinse cycles of the automatic dishwashing or laundry washing machine, the machine-cleaning composition being present in the wash or rinse water (aqueous wash liquor).

11. A method according to any of claims 8 to 10 wherein the washing machine may be at least partially loaded with articles during the at least one wash cycle or at least one rinse cycle.

12. A method according to any of claims 8 to 11 wherein the machine-cleaning composition is provided in two or more sequential wash and/or rinse cycles of the washing machine.

13. Use of a machine-cleaning composition according to any of claims 1 to 7 for cleaning washing machines such as laundry washing machines or dishwashing machines, either domestic or commercial/institutional.

Europäisches Patentamt
European Patent Office
Office européen des brevets

# EUROPEAN SEARCH REPORT

Application Number
EP 22 17 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/201403 A1 (NOVOZYMES AS [DK]) 8 October 2020 (2020-10-08) * the whole document * ----- | 1 | INV. C11D3/386 |
| Y | WO 2020/008043 A1 (NOVOZYMES AS [DK]) 9 January 2020 (2020-01-09) * paragraphs [0003], [0006], [0086], [0160], [4345]; claim 6 * ----- | 1 | |
| Y | US 2021/032570 A1 (LINDSAY JEFFREY DEAN [US]) 4 February 2021 (2021-02-04) * pages 19, 105 * ----- | 1 | |
| A | WO 2013/087043 A1 (DALIAN CHEMICAL PHYSICS INST [CN]) 20 June 2013 (2013-06-20) ----- | 4 | |
| A | CN 109 957 536 A (VLAND BIOTECH GROUP CO LTD; WEIFANG KDN BIOTECH CO LTD) 2 July 2019 (2019-07-02) ----- | 4 | |
| A | CN 110 904 084 A (OCEAN UNIV CHINA) 24 March 2020 (2020-03-24) ----- | 4 | TECHNICAL FIELDS SEARCHED (IPC) |
| A | KR 2012 0116612 A (UNIV KOREA RES & BUS FOUND [KR]) 23 October 2012 (2012-10-23) ----- | 4 | C11D |
| A | US 2006/128946 A1 (WEINER RONALD M [US] ET AL) 15 June 2006 (2006-06-15) ----- | 4 | |
| A | CN 112 980 822 A (QINGDAO INST BIOENERGY & BIOPROCESS TECH CAS) 18 June 2021 (2021-06-18) ----- | 4 | |
| A | "MULTISPECIES: polysaccharide lyase family 7 protein [Flavobacterium]", PROTEIN,, 28 July 2021 (2021-07-28), XP002805950, ----- | 4 | |
| | -/-- | | |

The present search report has been drawn up for all claims

1

| Place of search | Date of completion of the search | Examiner |
|---|---|---|
| The Hague | 14 October 2022 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 17 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "SubName: Full=Alginate lyase {ECO:0000313¦EMBL:SFE00983.1}", UNIPROT,, 22 November 2017 (2017-11-22), XP002805948, | 4 | |
| A | "polysaccharide lyase family 7 protein [Flavobacterium collinsii]", PROTEIN,, 31 July 2021 (2021-07-31), XP002805949, | 4 | |
| A | KELLY M. COLVIN ET AL: "The Pel and Psl polysaccharides provide Pseudomonas aeruginosa structural redundancy within the biofilm matrix", ENVIRONMENTAL MICROBIOLOGY, vol. 14, no. 8, 19 December 2011 (2011-12-19), pages 1913-1928, XP055295391, GB ISSN: 1462-2912, DOI: 10.1111/j.1462-2920.2011.02657.x | 1 | TECHNICAL FIELDS SEARCHED (IPC) |
| A | MICHAEL J. FRANKLIN ET AL: "Biosynthesis of the Pseudomonas aeruginosa Extracellular Polysaccharides, Alginate, Pel, and Psl", FRONTIERS IN MICROBIOLOGY, vol. 2, 1 January 2011 (2011-01-01), XP055295390, DOI: 10.3389/fmicb.2011.00167 | 1 | |

-/--

The present search report has been drawn up for all claims

1

| Place of search | Date of completion of the search | Examiner |
|---|---|---|
| The Hague | 14 October 2022 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 17 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BAKER PERRIN ET AL: "Exopolysaccharide biosynthetic glycoside hydrolases can be utilized to disrupt and prevent Pseudomonas aeruginosa biofilms", SCIENCE ADVANCES, vol. 2, no. 5, 6 May 2016 (2016-05-06), XP55970364, US ISSN: 2375-2548, DOI: 10.1126/sciadv.1501632 ----- | 1 | |
| A | US 6 486 112 B1 (BETTIOL JEAN-LUC PHILIPPE [BE] ET AL) 26 November 2002 (2002-11-26) * claims 1,4 * ----- | 1 | |
| A | CHENG DANYANG ET AL: "Characteristics and applications of alginate lyases: A review", INTERNATIONAL JOURNAL OF BIOLOGICAL MACROMOLECULES, ELSEVIER BV, NL, vol. 164, 31 July 2020 (2020-07-31), pages 1304-1320, XP086335593, ISSN: 0141-8130, DOI: 10.1016/J.IJBIOMAC.2020.07.199 [retrieved on 2020-07-31] ----- | 1 | TECHNICAL FIELDS SEARCHED (IPC) |
| A | PHILIPP STIEFEL ET AL: "Enzymes Enhance Biofilm Removal Efficiency of Cleaners", ANTIMICROBIAL AGENTS AND CHEMOTHERAPY, vol. 60, no. 6, 4 April 2016 (2016-04-04), pages 3647-3652, XP55495972, US ISSN: 0066-4804, DOI: 10.1128/AAC.00400-16 ----- | 1 | |
| A | WO 2020/070011 A1 (NOVOZYMES AS [DK]) 9 April 2020 (2020-04-09) * claim 3 * ----- -/-- | 1 | |

The present search report has been drawn up for all claims

1

| Place of search | Date of completion of the search | Examiner |
|---|---|---|
| The Hague | 14 October 2022 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 17 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 1 315 997 A (NOVO JYMES AS [DK]) 3 October 2001 (2001-10-03) * page 32, paragraph 2 * ----- | 1 | |
| A | US 2021/009922 A1 (KLINGMAN SHANNON E [US] ET AL) 14 January 2021 (2021-01-14) * paragraphs [0044], [0128], [0129] * ----- | 1 | |
| T | WO 2022/094164 A1 (PROCTER & GAMBLE [US]) 5 May 2022 (2022-05-05) ----- | | |

TECHNICAL FIELDS SEARCHED (IPC)

The present search report has been drawn up for all claims

1

| Place of search | Date of completion of the search | Examiner |
|---|---|---|
| The Hague | 14 October 2022 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 17 1692

This annex lists the patent family members relating to the patent documents cited in the above-mentioned European search report. The members are as contained in the European Patent Office EDP file on
The European Patent Office is in no way liable for these particulars which are merely given for the purpose of information.

14-10-2022

| Patent document cited in search report | Publication date | Patent family member(s) | Publication date |
|---|---|---|---|
| WO 2020201403 A1 | 08-10-2020 | CN 113785039 A | 10-12-2021 |
| | | EP 3947619 A1 | 09-02-2022 |
| | | US 2022169953 A1 | 02-06-2022 |
| | | WO 2020201403 A1 | 08-10-2020 |
| WO 2020008043 A1 | 09-01-2020 | EP 3818140 A1 | 12-05-2021 |
| | | US 2021253981 A1 | 19-08-2021 |
| | | WO 2020008043 A1 | 09-01-2020 |
| US 2021032570 A1 | 04-02-2021 | NONE | |
| WO 2013087043 A1 | 20-06-2013 | CN 102994407 A | 27-03-2013 |
| | | WO 2013087043 A1 | 20-06-2013 |
| CN 109957536 A | 02-07-2019 | NONE | |
| CN 110904084 A | 24-03-2020 | NONE | |
| KR 20120116612 A | 23-10-2012 | NONE | |
| US 2006128946 A1 | 15-06-2006 | NONE | |
| CN 112980822 A | 18-06-2021 | NONE | |
| US 6486112 B1 | 26-11-2002 | NONE | |
| WO 2020070011 A1 | 09-04-2020 | CN 112969775 A | 15-06-2021 |
| | | EP 3861094 A1 | 11-08-2021 |
| | | WO 2020070011 A1 | 09-04-2020 |
| CN 1315997 A | 03-10-2001 | AU 770911 B2 | 04-03-2004 |
| | | BR 9912158 A | 10-04-2001 |
| | | CA 2333491 A1 | 27-01-2000 |
| | | CN 1315997 A | 03-10-2001 |
| | | EP 1098964 A1 | 16-05-2001 |
| | | JP 2002520049 A | 09-07-2002 |
| | | KR 20010079542 A | 22-08-2001 |
| | | MX PA01000556 A | 14-05-2002 |
| | | WO 0004138 A1 | 27-01-2000 |
| US 2021009922 A1 | 14-01-2021 | NONE | |
| WO 2022094164 A1 | 05-05-2022 | WO 2022094163 A1 | 05-05-2022 |
| | | WO 2022094164 A1 | 05-05-2022 |
| | | WO 2022094588 A1 | 05-05-2022 |
| | | WO 2022094589 A1 | 05-05-2022 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 22 17 1692

This annex lists the patent family members relating to the patent documents cited in the above-mentioned European search report. The members are as contained in the European Patent Office EDP file on
The European Patent Office is in no way liable for these particulars which are merely given for the purpose of information.

14-10-2022

| Patent document cited in search report | Publication date | Patent family member(s) | Publication date |
|---|---|---|---|
| | | WO 2022094590 A1 | 05-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9839402 A
- WO 2004067737 A
- WO 2015091989 A
- WO 2015091990 A
- WO 2015024739 A
- WO 2015143360 A
- US 6312936 B1
- US 5679630 A
- US 4760025 A
- DE 102006022216 A1
- DE 102006022224 A1
- WO 2015089447 A
- WO 2015089441 A
- WO 2016066756 A
- WO 2016066757 A
- WO 2016069557 A
- WO 2016069563 A
- WO 2016069569 A
- WO 2016174234 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- WO 0037627 A
- WO 08010925 A
- US 5352604 A
- US 6939702 B1
- US PA20090217464 A
- WO 2013171241 A
- WO 2011084412 A
- WO 2013033318 A
- WO 2018228880 A
- WO 2018228881 A
- EP 3299457 A
- WO 2018209026 A
- WO 2017036901 A
- WO 2017005798 A
- US 4435307 A
- US 5648263 A
- US 5691178 A
- US 5776757 A
- US 7141403 B2
- WO 09148983 A
- WO 2017106676 A
- US 7153818 B
- WO 9700324 A
- EP 1022334 A
- US 5856164 A
- WO 9923211 A
- WO 9623873 A
- WO 0060060 A
- WO 06002643 A
- WO 2017192657 A
- WO 2011100410 A
- WO 2013003659 A
- US 6093562 A
- WO 09149130 A
- WO 10115021 A
- WO 2016091688 A
- WO 2014099523 A
- WO 2014164777 A
- WO 2009149271 A
- WO 2016180748 A
- WO 2018060216 A
- WO 9324618 A
- WO 9510602 A
- WO 9815257 A
- WO 2018191135 A
- WO 2015040159 A
- WO 2017021515 A
- WO 2017021516 A
- WO 2017021517 A
- WO 2019081515 A
- WO 2017162836 A
- WO 2018108865 A
- WO 2018011277 A
- EP 3476935 A
- WO 2018178061 A
- WO 2020074499 A
- WO 2018184873 A
- WO 2017186936 A
- WO 2017186937 A
- WO 2017186943 A
- WO 2017207770 A
- WO 2019086520 A
- WO 2019086528 A
- WO 2019086530 A
- WO 2019086532 A
- WO 2019086521 A
- WO 2019086526 A
- WO 2020002604 A
- WO 2020002608 A

- WO 2020007863 A
- WO 2020007875 A
- WO 2020008024 A
- WO 2020070063 A
- WO 2020070249 A
- WO 2020088957 A
- WO 2020088958 A
- WO 2020207944 A
- WO 2013167581 A
- WO 2015181299 A
- WO 2015181292 A
- WO 2017046232 A
- WO 2017046260 A
- WO 201837062 A
- WO 201837065 A
- WO 2019038059 A
- WO 2019162000 A
- WO 2015001017 A
- WO 2018037061 A
- WO 201837064 A
- WO 2019038060 A
- WO 2019038057 A
- WO 2015185689 A
- WO 09118375 A
- WO 9813459 A

**Non-patent literature cited in the description**


- **NEEDLEMAN ; WUNSCH.** *J. Mol. Biol.,* 1970, vol. 48, 443-453
- **RICE et al.** *Trends Genet.,* 2000, vol. 16, 276-277
- **DARTOIS et al.** *Biochemica et Biophysica Acta,* 1993, vol. 1131, 253-360